(19) Europäisches Patentamt
European Patent Office
Office européen des brevets

(11) **EP 3 876 913 B1**

(12) **EUROPEAN PATENT SPECIFICATION**

(45) Date of publication and mention
of the grant of the patent:
**11.03.2026  Bulletin 2026/11**

(21) Application number: **19805748.1**

(22) Date of filing: **05.11.2019**

(51) International Patent Classification (IPC):
*A61K 9/00* (2006.01)        *A61K 9/51* (2006.01)
*A61K 47/28* (2006.01)       *A61K 47/44* (2017.01)
*A61P 27/04* (2006.01)

(52) Cooperative Patent Classification (CPC):
**A61K 9/0048; A61K 9/5123; A61K 47/28;**
**A61K 47/44; A61P 27/04**

(86) International application number:
**PCT/IB2019/059479**

(87) International publication number:
**WO 2020/095192 (14.05.2020 Gazette 2020/20)**

(54) **ARTIFICIAL TEARS**

KÜNSTLICHE TRÄNEN

LARMES ARTIFICIELLES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:   **05.11.2018   GB 201818043**

(43) Date of publication of application:
**15.09.2021   Bulletin 2021/37**

(73) Proprietor: **Waterford Institute Of Technology**
**Waterford (IE)**

(72) Inventors:
• **FITZHENRY, Laurence**
**Waterford Institute of Technology Cork Road**
**Waterford (IE)**
• **KUMARI, Sangeeta**
**Waterford Institute of Technology Cork Road**
**Waterford (IE)**

(74) Representative: **Barker Brettell LLP**
**100 Hagley Road**
**Edgbaston**
**Birmingham, West Midlands B16 8QQ (GB)**

(56) References cited:
WO-A1-2015/168523        WO-A1-2019/123417
WO-A1-2019/123420        WO-A2-2011/116963
US-A1- 2014 080 897

• **GONZALEZ-MIRA E ET AL: "Optimizing**
**flurbiprofen-loaded NLC by central composite**
**factorial design for ocular delivery;Optimizing**
**flurbiprofen-loaded NLC by central composite**
**factorial design for ocular delivery",**
**NANOTECHNOLOGY, INSTITUTE OF PHYSICS**
**PUBLISHING, BRISTOL, GB, vol. 22, no. 4, 20**
**December 2010 (2010-12-20), pages 45101,**
**XP020202770, ISSN: 0957-4484, DOI: 10.1088/**
**0957-4484/22/4/045101**
• **QIANWEN LI ET AL: "A Review of the Structure,**
**Preparation, and Application of NLCs, PNPs, and**
**PLNs", NANOMATERIALS, vol. 7, no. 6, 27 May**
**2017 (2017-05-27), pages 122, XP055504982, DOI:**
**10.3390/nano7060122**

**Description**

The present invention relates as defmed by the claims to Nanostructured Lipid Carrier (NLC) particle for ocular delivery, and associated compositions, uses and treatments.

Dry eye syndrome (keratoconjunctivitis sicca) is a common complaint, with a number of potential causes. It is characterised by a chronic lack of sufficient lubrication and moisture on the surface of the eye, leading to a breakdown of the tear film. There are three main components to the tear film: oil, water and mucin each provided by different glands present on or around the eye. Imbalance between these three components, e.g. due to dysfunction of one or more of the glands, results in an insufficient tear film and the symptoms of dry eye.

One of the major causes of dry eye syndrome is Meibomian Gland Dysfunction (MGD). Meibomian glands are small glands found in the upper and lower eyelids, which function to secrete oils onto the surface of the eye, reducing the evaporation rate of tears. Dysfunction of the Meibomian glands, through blockage or another abnormality, leads to a reduction of oil secretion and a subsequent increase in the evaporation rate of tears, thus leading to dry eye syndrome.

One of the major challenges for the pharmacologist and formulation scientist is ocular delivery. The eye is complex with unique physiology and anatomy, and it is considered to be a protective organ that is highly impervious to foreign substances. The eye has a lacrimation mechanism, which impedes the bioavailability of formulations such as eye drops. The eye structural barrier allows only less than 5% of a typical formulation to enter the eye. Therefore, it's challenging for formulation scientists to develop eye drops that can overcome this obstacle.

There are several artificial tear-based eye drops available on the market. Artificial tears provide a soothing effect for dry eye syndrome. These are lubricating eye drops used to treat the dryness and irritation associated with deficient tear production in keratoconjunctivitis sicca. One of the examples of non-medicated emulsion based lubricating eye drop is Refresh Endura®. As discussed above, most of the eye drop is less bioavailable due to the lacrimation, causing a reduction in therapeutic effect. To overcome this problem frequent dosing is required to achieve the therapeutic concentration.

The marketed emulsions also face instability, such as coalescence, flocculation and creaming. To prevent this instability they are typically formulated with surfactant, which results in increasing the kinetic stability of the emulsion. However, the addition of surfactant to emulsion is associated with cytotoxicity. In particular, it has been reported that suspension based formulations show disadvantages such as additive toxicity and blurred vision. To overcome these challenges a safer and more effective artificial tear composition is needed.

According to the first aspect of the invention, there is provided a Nanostructured Lipid Carrier (NLC) particle, wherein the Nanostructured Lipid Carrier particle comprises:

(i) a solid outer shell comprising a solid lipid, wherein the solid lipid comprises cholesterol; and
(ii) a liquid core comprising a liquid lipid, wherein the liquid lipid comprises triglyceride, wherein the NLC particle does not comprise or encapsulate any additional therapeutic agent.

The NLC particles according to the invention are advantageously nontoxic, biocompatible, and show favourable mucoadhesion, which result in an increase of residence time of the NLC particle on the cornea. The triglyceride, such as castor oil or oils of similar triglyceride component, can advantageously provide anti-inflammatory properties. Advantageously, it can act as a penetration enhancer, aiding the transportation of the NLC particles according to the invention transcellularly to the cornea. The triglyceride, such as castor oil or oils of similar triglyceride component, can also reduce the evaporation of tears from the eye and may be used to treat dry eye disorders such as Meibomian gland dysfunction, due to their anti-inflammatory action. As cell membrane consists of lipid bilayer, the use of cholesterol as the solid lipid component will advantageously provide better diffusion of the NLC through the cell membrane.

In one embodiment, the triglyceride comprises castor oil triglyceride. In particular, the liquid core may comprise or consist of castor oil. In another embodiment, the liquid core may comprise or consist of castor oil triglyceride or an equivalent triglyceride having substantially similar fatty acid content as castor oil triglyceride.

In one embodiment, the triglyceride comprises omega-3-fatty acid. In particular, the liquid core may comprise of consist of omega-3-fatty acid. In another embodiment, the liquid core may comprise or consist of omega-3-fatty acid triglyceride or an equivalent triglyceride having substantially similar fatty acid content as omega-3-fatty acid triglyceride.

In one embodiment, the triglyceride comprises ricinoleic acid as a majority fatty acid component. In one embodiment, the triglyceride comprises at least 85% ricinoleic acid. The triglyceride may comprise ricinoleic acid, oleic acid and linoleic acid. In one embodiment, the triglyceride comprises about 85%-95% ricinoleic acid, about 2%-6% oleic acid and about 1%-5% linoleic acid. Additionally or alternatively, the triglyceride may comprise ricinoleic acid, oleic acid, linoleic acid, alpha-linolenic acid, stearic acid, palmitic acid and dihydroxystearic acid.

In one embodiment, NLC particles according to the invention are suitable for treatment or prevention of a dry eye disorder. The dry eye disorder may comprise any one of the disorders selected from dry eye syndrome (keratoconjunctivitis sicca); conjunctivitis; keratitis; uveitis; sceritis; episcleritis; blepharitis; and iritis; or combinations thereof. In one embodiment, the therapeutic agent is suitable for treatment or prevention of dry eye.

In one embodiment, the NLC particle does not encapsulate or comprise a therapeutic agent other than the liquid lipid, such as triglyceride, which may itself be considered to have therapeutic properties, such as anti-inflammatory properties. In another embodiment, the NLC particle does not encapsulate or comprise a therapeutically effective amount of a therapeutic agent. In another embodiment, the NLC particle does not encapsulate or comprise a steroid, such as dexamethasone. Additionally or alternatively, the NLC particle does not encapsulate or comprise a steroid or an antibiotic. Additionally or alternatively, the NLC particle does not encapsulate or comprise one or more, or any of the drugs selected from an anti-cancer drug (such as docetaxel or irinotecan), antifungal drug (such as amphotericin B), nutraceutical drug/agent (such as curcumin, resveratrol, honokiol/magnolol, or quercetin). Additionally or alternatively, the NLC particle may not encapsulate or comprise a chalcone, or a therapeutically effective amount of a chalcone. In one embodiment, the chalcone comprises isoliquiritigenin (ILTG). In one embodiment the NLC particle does not encapsulate or comprise an anti-cancer drug.

In one embodiment, the NLC particle is not a liposome. The NLC particle may not encapsulate or comprise lecithin and/or vitamin A.

The Nanostructured Lipid Carrier particle may be of nanoparticle size. In one embodiment, the Nanostructured Lipid Carrier particle is less than about 1000nm at its largest diameter. In another embodiment, the Nanostructured Lipid Carrier particle is less than about 900nm at its largest diameter. In another embodiment, the Nanostructured Lipid Carrier particle is less than about 800nm at its largest diameter. In another embodiment, the Nanostructured Lipid Carrier particle is less than about 700nm at its largest diameter. In another embodiment, the Nanostructured Lipid Carrier particle is less than about 600nm at its largest diameter. In another embodiment, the Nanostructured Lipid Carrier particle is less than about 500nm at its largest diameter. In another embodiment, the Nanostructured Lipid Carrier particle is less than about 400nm at its largest diameter. In another embodiment, the Nanostructured Lipid Carrier particle is less than about 300nm at its largest diameter. In another embodiment, the Nanostructured Lipid Carrier particle is less than about 200nm at its largest diameter. In another embodiment, the Nanostructured Lipid Carrier particle is less than about 100nm at its largest diameter. In another embodiment, the Nanostructured Lipid Carrier particle is less than about 50nm at its largest diameter. In another embodiment, the Nanostructured Lipid Carrier particle is between about 5 nm and about 1000nm at its largest diameter. In another embodiment, the Nanostructured Lipid Carrier particle is between about 5nm and about 800nm at its largest diameter. In another embodiment, the Nanostructured Lipid Carrier particle is between about 5nm and about 500nm at its largest diameter. In another embodiment, the Nanostructured Lipid Carrier particle is between about 5 nm and about 100nm at its largest diameter. In another embodiment, the Nanostructured Lipid Carrier particle is between about 5nm and about 50nm at its largest diameter. In another embodiment, the Nanostructured Lipid Carrier particle is between about 5nm and about 30nm at its largest diameter. In another embodiment, the Nanostructured Lipid Carrier particle is between about 5nm and about 21nm at its largest diameter. In another embodiment, the Nanostructured Lipid Carrier particle may be about 304nm at its largest diameter. The Nanostructured Lipid Carrier particle may be substantially spherical in shape.

In one embodiment, the NLC particle comprises about 1:1 of solid outer shell relative to liquid core. The percentage v/w of liquid core of an NLC particle may be an average in a population of NLCs.

In one embodiment, the NLC particle comprises about 1:1 of cholesterol relative to castor oil. Alternatively, the NLC particle comprises about 1:0.5 of cholesterol relative to castor oil. Alternatively, the NLC particle comprises about 1:1.5 of cholesterol relative to castor oil. In another embodiment, the NLC particle comprises about 1:0.5-1.5 of cholesterol relative to castor oil. In another embodiment, the NLC particle comprises about 1:0.8-1.2 of cholesterol relative to castor oil. The percentage v/w of liquid core of an NLC particle may be an average in a population of NLCs.

According to another aspect of the invention, there is provided a composition comprising a plurality of NLC particles according to the invention herein.

The composition may be a pharmaceutically acceptable composition. The composition may be an ophthalmically acceptable composition. For example, the NLC particles may be provided in a pharmaceutically acceptable carrier. The pharmaceutically acceptable carrier may be an ophthalmically acceptable carrier. The composition may be suitable for topical administration to the eye. In one embodiment, the composition is an ophthalmic composition. An ophthalmic composition is understood to be a sterile, liquid, semi-solid, or solid preparation that may or may not contain one or more active pharmaceutical ingredient(s) (i.e. the anti-inflammatory castor oil described herein) intended for application to the eye or eyelid.

The composition may be in the form of the NLC particles suspended in a gel, lotion, cream, ointment, or solution, such as an aqueous solution. In one embodiment, the composition is in the form of an eye drop formulation.

The composition may comprise one or more ophthalmically acceptable ingredients selected from the group consisting of: water; saline; salt; buffer; demulcent; humectant; viscosity increasing agent; tonicity adjusting agent; cellulose derivatives e.g. carboxymethylcellulose sodium, hydroxyethyl cellulose, hydroxypropyl methylcellulose, or methylcellulose; dextran 70; gelatin; polyols; glycerine; polyethylene glycol e.g. PEG300 or PEG400; polysorbate 80; propylene glycol; polyvinyl alcohol; and povidone (polyvinyl pyrrolidone); and combinations thereof.

Demulcents may comprise or consist of cellulose derivatives, glycerine, polyvinyl alcohol, polyvinyl pyrrolidone, cellulose derivatives, polyethylene glycol, or combinations thereof.

In one embodiment, the carrier of the NLC particles in the composition, does not substantially comprise a surfactant, such as polysorbate/Tween 80. In one embodiment, the carrier of the NLC particles in the composition, does not comprise a physiologically relevant concentration of surfactant, such as polysorbate/Tween 80. In one embodiment, the carrier of the NLC particles in the composition comprises no more than 0.05% or no more than 1% of a surfactant, such as polysorbate/Tween 80. In one embodiment, the carrier of the NLC particles in the composition comprises no more than 1.5% of a surfactant, such as polysorbate/Tween 80.

According to another aspect of the present invention, there is provided a composition according to the invention herein for use in the treatment or prevention of a dry eye disorder in a subject.

According to another aspect of the present invention, there is provided the use of a composition according to the invention herein in the manufacture of a medicament for treatment or prevention of a dry eye disorder in a subject.

According to another aspect of the present invention, there is provided a method of treatment or prevention of a dry eye disorder in a subject comprising the administration of the composition according to the invention to an eye of the subject.

The administration may be topical to the surface of the eye or to the eyelid.

The subject may be mammalian. In one embodiment, the subject is a human subject. The subject may be in need of treatment for the dry eye disorder or may be at risk of developing the dry eye disorder.

The treatment or prevention may comprise a single administration or repeated administrations. The administration may be once every day, or at least once per day. The administration may be once every 2 days. The administration may be once every 1 to 7 days. The administration may be twice every day. The administration may be more than twice every day. The administration may be three or more times a day.

The dry eye disorder may be selected from dry eye syndrome (keratoconjunctivitis sicca), conjunctivitis, keratitis, uveitis, scelritis, episcleritis, blepharitis, and iritis; or combinations thereof. The disorder may be acute or chronic.

In another aspect of the invention, there is provided an eye drop dispenser or eye wash device comprising the composition according to the invention herein.

An eye drop dispenser may otherwise be known as an eye drop applicator. Typical eye drop dispensers comprise a reservoir for the composition and an outlet for the composition. The outlet may be tapered towards a distal end, with the outlet orifice at the tip/distal end. The dispenser may be arranged to be sealed, for example with a cap. An eye drop dispenser may alternatively comprise a syringe device.

The skilled person will be familiar with the term "Nanostructured Lipid Carrier (NLC)" where it is intended to an artificial tear composed of both solid and liquid lipids as a core matrix.

The term "solid" in the context of the solid outer shell is understood to mean solid at room temperature (i.e. about 24°C). i.e. the outer shell is solid at room temperature. The term "liquid" in the context of the liquid core is understood to mean liquid at room temperature (i.e. about 24°C). i.e. the core is liquid at room temperature. In one embodiment, the solid outer shell of the NLC particles is solid at body temperature (e.g. 37°C).

The term "solid" in the context of the solid lipid is understood to mean a composition of the lipid is a solid at room temperature (i.e. about 24°C), i.e. the lipid exists as a solid at room temperature. In one embodiment, the solid lipid forms a solid at body temperature (e.g. 37°C). The term "liquid" in the context of the liquid lipid is understood to mean a composition of the lipid is a liquid at room temperature (i.e. about 24°C), i.e. the lipid exists as a liquid at room temperature.

The term "prevention" means avoidance of a disorder or a protective treatment for a disorder. The prevention may include a reduced risk of the disorder, reduced risk of infection, transmission and/or progression, or reduced severity of the disorder.

The term "treatment" means a cure of a condition or disease, an alleviation of symptoms, or a reduction in severity of a disorder or symptoms of the disorder.

The term "dry eye disorder" used herein is understood to mean any condition in which the typical symptoms associated with dry eyes, such as feelings of dryness, grittiness or soreness that worsens throughout the day, itchy, scratchy, stinging or tired eyes possibly accompanied by pain and/or redness. For example, conditions such as dry eye syndrome (keratoconjunctivitis sicca), meibomian gland dysfunction, or dry eyes associated with the use of contact lenses or certain medications.

The term "therapeutic agent", "therapeutic active", or "active agent" used herein is understood to mean any small molecule or biological drug that is arranged to directly bind to cells, cellular components, cell membrane, nucleic acids or proteins such as enzymes, and that are intended to activate or inhibit a biological pathway or other cellular process. In one embodiment, the solid lipid and/or liquid lipid (e.g. cholesterol and/or triglyceride respectively) may not be considered a small molecule or biological drug/therapeutic agent. In another embodiment, the solid lipid and/or liquid lipid (e.g. cholesterol and/or triglyceride respectively) may be considered to have therapeutic effects, but the NLC may not comprise a further drug/therapeutic agent.

The skilled person will understand that optional features of one embodiment or aspect of the invention may be applicable, where appropriate, to other embodiments or aspects of the invention.

Embodiments of the invention will now be described in more detail, by way of example only, with reference to the accompanying drawings.

**Figure 1.** Cytotoxicity study of Cholesterol:Castor oil-NLCs (CHCAO-NLC) with MTT assay (colorimetric assay for assessing cell metabolic activity).

**Figure 2.** Cellular uptake study of CHCAO-NLC with fluorescence microscopy.

**Figure 3.** Ex vivo mucoadhesion study with fluorescence microscopy. Porcine eye cornea was incubated with CHCAO-NLCs for four hours and then examined at 10x magnification. (a) control in DIC mode; (b) control on FITC mode; (c) CHCAO-NLCs treated in DIC mode; and (d) CHCAO-NLCs in FITC mode.

**Figure 4.** MMP-9 marker study of CHCAO-NLCs. C - negative control; C+ L - positive control with LPS; C+L+D0.1 or 0.5 - cells with varying concentrations ($\mu$g/ml) of CHCAO-NLCs.

## Example 1- Size measurement and zeta potential measurement:

The particle size and zeta potential NLCs is characterised by DLS. The characterisation showed that sample with Cholesterol - Castor Oil nanostructured lipid carrier (CHCACO-NLCs) ratio 1:1 is the most stable compared to other ratios. The size and zeta potential of CHCAO-NLCs is given in Table 1. Sample Particle size (nm) Zeta potential (mV)

**Table 1.** Size and zeta potential of CHCAO-NLCs.

| SAMPLE NAME | PARTICLE SIZE (nm) | ZETA POTENTIAL (mV) |
| --- | --- | --- |
| CHCAO 1:1 | 304 | -8.5 |

## Example 2- Cytotoxicity Assessment of the prepared CHCACO-NLCs:

The cytotoxicity of the CHCAO-NLCs was carried out on Human Corneal Epithelium Cells (HCEC). The cells with the initial density of 10,000 cells/well were seeded in a 96-well plate and were then cultured for 24 h in DMEM medium containing 10% FBS.

The cells were then treated with varying concentrations of CHCAO-NLCs, and CHCAO-NLCs treated cells were incubated in a humidified environment with 5% CO2 at 37 °C for 4 h. After 4 h culture medium is replaced with the fresh medium, the cells were further maintained for another 20 h. After the specified time, MTT (3-(4,5-dimethylthiazol-2-yl)-2,5-diphenyltetrazolium bromide) reagent was added to each well, and the cells were incubated for another 4 h. The medium in each well was replaced with 200$\mu$L of DMSO to dissolve the formazan crystals. The absorbance (O.D.) was recorded with a microplate reader. The cell viability was calculated by using the following equation:

$$\text{Cell viability (\%)} = [\text{O.D. (test)}/\text{O.D. (control)}] \times 100$$

Where O.D. (test) and O.D. (control) are the absorbance values of the cells cultured with and without NLC, respectively.

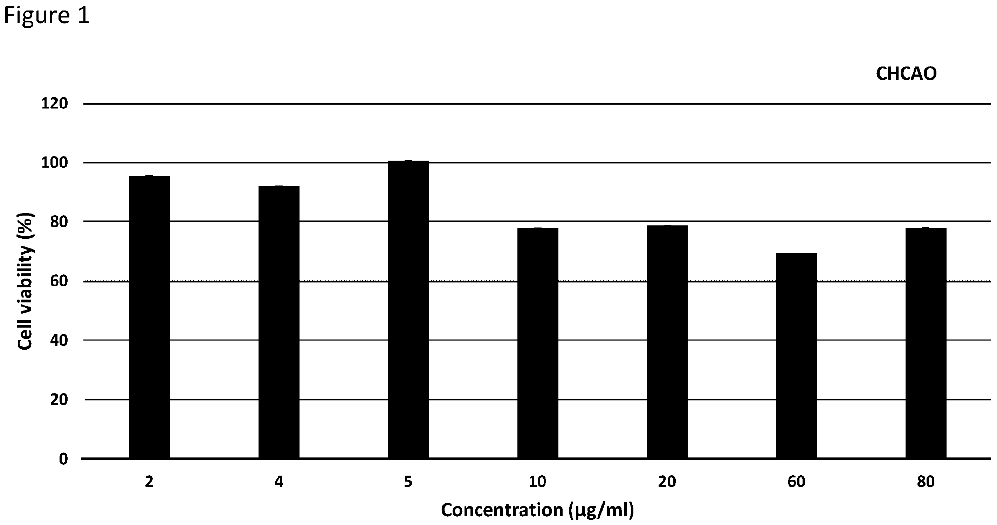

The cytotoxicity study showed that prepared 1:1 ratio CHCAO-NLCs is non-toxic up to the given concentration **Figure 1.**

## Example 3- Cellular Uptake study

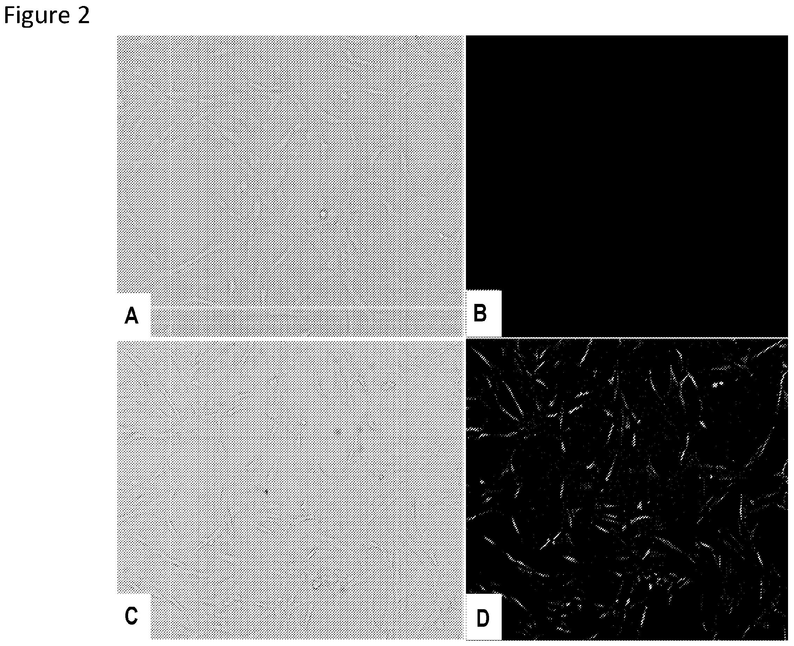

In vitro cellular uptake of coumarin-6 labelled CHCAO-NLCs was further examined using fluorescent microscopy. The cells were seeded in confocal imaging dishes at a density of $5 \times 10^4$ cells per dish. The coumarin-6 labelled CHCAO-NLCs were prepared and were exposed to the HCEC cells in medium. After 4 h of incubation at 37 °C, CHCAO-NLCs treated medium is removed and the cells are washed. Further cellular uptake study is performed under fluorescent microscope. The data showed that NLCs are internalised in the cell and the coumarin-6, which is encapsulated in CHCAO-NLCs is successfully released in the cytoplasm of the HCEC cells. Indicating that dexamethasone encapsulated in CHCAO-NLCs will be released within the HCEC cells **(Figure 2).**

**Example 4- Mucoadhesion study**

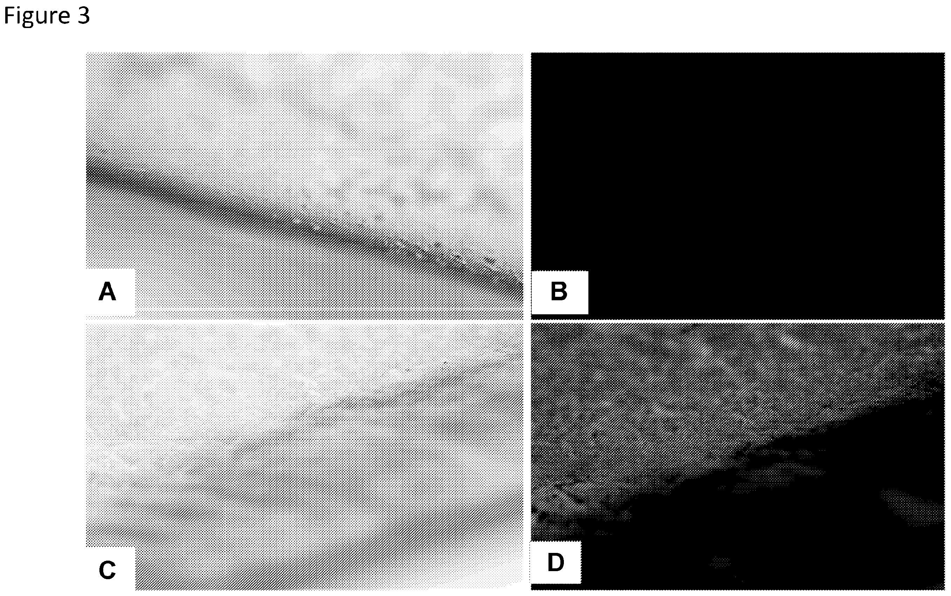

Mucoadhesion study was performed ex vivo with pig cornea. The mucoadhesion study of coumarin-6 labelled CHCAO-NLCs on pig cornea was further examined using fluorescent microscopy. The pig cornea is excised and placed in the confocal imaging dishes containing medium. The coumarin-6 labelled CHCAO-NLCs were prepared and were exposed to the cornea in medium. After 4 h of incubation at 37 °C, CHCAO-NLCs treated medium is removed and the cornea is washed. Further mucoadhesion study is performed under fluorescent microscope. The mucoadhesion study showed that the CHCAO-NLCs are mucoadhesive. As the CHCAO-NLCs particles stick to the cornea **(Figure 3)**.

**Example 5- Marker Study**

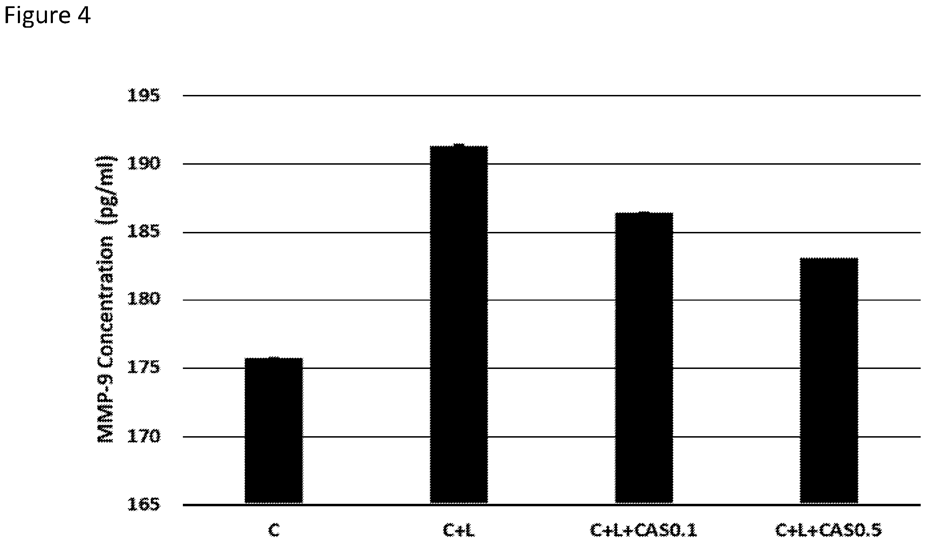

The marker study is performed for MMP-9 protein. $5x10^4$ cells are seeded in 6 well plate containing DMEM media. As the confluency reached 70% cells are transfected with lipopolysaccharide and incubated for 24h in a humidified environment with 5% CO2 at 37 °C. After 24h medium is replaced with the fresh media and cells are transfected with varying concentration of the CHCAO-NLCs and incubated for 24h. Protein is extracted from the cells after 24h and used for Elisa. Elisa is performed to measure the MMP-9 concentration. Decrease in the concentration of MMP-9 marker has been observed **(Figure 4)**.

**Claims**

1.    A Nanostructured Lipid Carrier (NLC) particle comprising:

    (i) a solid outer shell comprising a solid lipid, wherein the solid lipid comprises cholesterol; and
    (ii) a liquid core comprising a liquid lipid, wherein the liquid lipid comprises triglyceride,

    wherein the NLC particle does not comprise or encapsulate any additional therapeutic agent.

2.    The NLC particle according to claim 1, wherein the triglyceride comprises ricinoleic acid as a majority fatty acid component.

3.    The NLC particle according to claim 1 or claim 2, wherein the triglyceride comprises at least 85% ricinoleic acid.

4.    The NLC particle according to any previous claim, wherein the triglyceride comprises ricinoleic acid, oleic acid and linoleic acid; or wherein the triglyceride is castor oil triglyceride.

5.    The NLC particle according to claim 1, wherein the NLC particle is suitable for treatment or prevention of a dry eye disorder.

6.    The NLC particle according to any previous claim, wherein the dry eye disorder comprises any one of the disorders selected from dry eye syndrome (keratoconjunctivitis sicca); conjunctivitis; keratitis; uveitis; scleritis; episcleritis; blepharitis; Meibomian gland dysfunction; and iritis; or combinations thereof.

7.    The NLC particle according to any preceding claim, wherein the NLC particle is nanoparticle size.

8.    The NLC particle according to any preceding claim, wherein the NLC particle comprises 1:1 w/w of solid outer shell relative to liquid core.

9.    The NLC particle according to any of claims preceding claim, wherein the NLC particle comprises 1:1 w/w of cholesterol relative to castor oil.

10.    A composition comprising a plurality of NLC particles in accordance with any preceding claim.

11.    The composition of claim 10, wherein the composition does not comprise any additional therapeutic active agent.

12.    The composition according to any one of claims 10 or 11, wherein the composition is an ophthalmically acceptable composition.

**13.** The NLC particle according to any one of claims 1 to 9, or the composition according to any one of claims 10 to 12, for use in the treatment or prevention of a dry eye disorder in a subject.

**14.** An eye drop dispenser or eye wash device comprising the NLC particle according to any one of claims 1 to 9, or the composition according to any one of claims 10 to 12.

**Patentansprüche**

**1.** Nanostrukturierter Lipidträgerpartikel (NLC-Partikel), umfassend:

(i) eine feste Außenhülle, die ein festes Lipid umfasst, wobei das feste Lipid Cholesterol umfasst; und
(ii) einen flüssigen Kern, der ein flüssiges Lipid umfasst, wobei das flüssige Lipid Triglycerid umfasst,

wobei der NLC-Partikel kein zusätzliches Therapeutikum umfasst oder einkapselt.

**2.** NLC-Partikel nach Anspruch 1, wobei das Triglycerid Rizinolsäure als eine Hauptfettsäurekomponente umfasst.

**3.** NLC-Partikel nach Anspruch 1 oder Anspruch 2, wobei das Triglycerid mindestens 85 % Rizinolsäure umfasst.

**4.** NLC-Partikel nach einem vorhergehenden Anspruch, wobei das Triglycerid Rizinolsäure, Oleinsäure und Linolsäure umfasst; oder wobei das Triglycerid Rizinusöltriglycerid ist.

**5.** NLC-Partikel nach Anspruch 1, wobei der NLC-Partikel zur Behandlung oder Prävention einer Störung mit trockenem Auge geeignet ist.

**6.** NLC-Partikel nach einem vorhergehenden Anspruch, wobei die Störung mit trockenem Auge eine der Störungen ausgewählt aus trockenem Auge (Kratoconjunctivitis sicca); Konjunktivitis; Keratitis; Uveitis; Skleritis; Episcleritis; Blepharitis; Meibom-Drüsen-Dysfunktion und Iritis; oder Kombinationen daraus umfasst.

**7.** NLC-Partikel nach einem vorhergehenden Anspruch, wobei der NLC-Partikel die Größe eines Nanopartikels aufweist.

**8.** NLC-Partikel nach einem vorhergehenden Anspruch, wobei der NLC-Partikel 1:1 Gew.-% an fester Außenhülle relativ zum flüssigen Kern umfasst.

**9.** NLC-Partikel nach einem vorhergehenden Anspruch, wobei der NLC-Partikel 1:1 Gew.-% an Cholesterol relativ zu Rizinusöl umfasst.

**10.** Zusammensetzung, umfassend eine Vielzahl von NLC-Partikeln gemäß einem vorhergehenden Anspruch.

**11.** Zusammensetzung nach Anspruch 10, wobei die Zusammensetzung keinen zusätzlichen therapeutischen Wirkstoff umfasst.

**12.** Zusammensetzung nach einem der Ansprüche 10 bis 11, wobei die Zusammensetzung eine ophthalmisch annehmbare Zusammensetzung ist.

**13.** NLC-Partikel nach einem der Ansprüche 1 bis 9 oder Zusammensetzung nach einem der Ansprüche 10 bis 12 zur Verwendung bei der Behandlung oder Prävention einer Störung mit trockenem Auge in einem Subjekt.

**14.** Augentropfenabgabevorrichtung oder Augenwaschvorrichtung, umfassend den NLC-Partikel nach einem der Ansprüche 1 bis 9 oder die Zusammensetzung nach einem der Ansprüche 10 bis 12.

**Revendications**

**1.** Particule de vecteur lipidique nanostructuré (NLC) comprenant :

(i) une coque externe solide comprenant un lipide solide, dans laquelle le lipide solide comprend du cholestérol ; et

(ii) un noyau liquide comprenant un lipide liquide, dans laquelle le lipide liquide comprend un triglycéride,

dans laquelle la particule de NLC ne comprend ni n'encapsule aucun agent thérapeutique supplémentaire.

2. Particule de NLC selon la revendication 1, dans laquelle le triglycéride comprend de l'acide ricinoléique en tant que composant d'acide gras majoritaire.

3. Particule de NLC selon la revendication 1 ou la revendication 2, dans laquelle le triglycéride comprend au moins 85 % d'acide ricinoléique.

4. Particule de NLC selon l'une quelconque des revendications précédentes, dans laquelle le triglycéride comprend de l'acide ricinoléique, de l'acide oléique et de l'acide linoléique ; ou dans laquelle le triglycéride est un triglycéride d'huile de ricin.

5. Particule de NLC selon la revendication 1, dans laquelle la particule de NLC est appropriée pour le traitement ou la prévention d'un trouble de la sécheresse oculaire.

6. Particule de NLC selon l'une quelconque des revendications précédentes, dans laquelle le trouble de la sécheresse oculaire comprend l'un quelconque des troubles choisis parmi le syndrome de l'œil sec (kératoconjonctivite sèche) ; la conjonctivite ; la kératite ; l'uvéite ; la sclérite ; l'épisclérite ; la blépharite ; un dysfonctionnement des glandes de Meibomius ; et l'iritis ; ou leurs combinaisons.

7. Particule de NLC selon l'une quelconque des revendications précédentes, dans laquelle la particule de NLC est de taille nanoparticulaire.

8. Particule de NLC selon l'une quelconque des revendications précédentes, dans laquelle la particule de NLC comprend un rapport p/p de 1:1 de la coque externe solide par rapport au noyau liquide.

9. Particule de NLC selon l'une quelconque des revendications précédentes, dans laquelle la particule de NLC comprend un rapport p/p de 1:1 de cholestérol par rapport à l'huile de ricin.

10. Composition comprenant une pluralité de particules de NLC conformément à l'une quelconque des revendications précédentes.

11. Composition selon la revendication 10, dans laquelle la composition ne comprend aucun agent actif thérapeutique supplémentaire.

12. Composition selon l'une quelconque des revendications 10 ou 11, dans laquelle la composition est une composition ophtalmiquement acceptable.

13. Particule de NLC selon l'une quelconque des revendications 1 à 9, ou composition selon l'une quelconque des revendications 10 à 12, pour une utilisation dans le traitement ou la prévention d'un trouble de la sécheresse oculaire chez un sujet.

14. Distributeur de gouttes oculaires ou dispositif de lavage oculaire comprenant la particule de NLC selon l'une quelconque des revendications 1 à 9, ou la composition selon l'une quelconque des revendications 10 à 12.

Figure 1

Figure 2

Figure 3

Figure 4

EP 3 876 913 B1